# EUROPEAN PATENT APPLICATION

(11) **EP 1 621 527 A1**
(43) Date of publication of application: **01.02.2006**
(21) Application number: 04722712.9
(22) Date of filing: 23.03.2004
(51) Int. Cl.: C07C 1/24, C07C 15/44, C07D 301/19, C07D 303/04

(54) **METHOD FOR PRODUCING ALPHA-METHYLSTYRENE**

(30) Priority: 26.03.2003 JP 2003085100
(71) Applicant: Sumitomo Chemical Company, Limited, Tokyo 104-8260 (JP)
(72) Inventor: TSUJI, Junpei, Ichihara-shi, Chiba 2990125 (JP); ISHINO, Masaru, Sodegaura-shi, Chiba 2990245 (JP)
(74) Representative: Lips, Hendrik Jan George
(86) International application number: PCT/JP2004/003971
(87) International publication number: WO 2004/085351

(57) **Abstract**

A process for producing α-methyl styrene from cumyl alcohol via dehydration in the presence of an activated alumina, **characterized in that** a concentration of an organic acid contained in a raw material containing cumyl alcohol is 10 to 1000 ppm by weight.

## Description

### TECHNICAL FIELD

The present invention relates to a process for producing α-methyl styrene.

### BACKGROUND ART

There is publicly known a process for producing α -methyl styrene by dehydrating cumyl alcohol in the presence of an activated alumina(e.g. JP52(1977)-39017 B). However, the conventional technique was not necessarily satisfied from the viewpoint of efficient attainment of high conversion of cumyl alcohol at low cost because it required considerably high temperature for attaining high conversion.

### DISCLOSURE OF THE INVENTION

The present invention is to provide a process for producing α-methyl styrene, which can efficiently attain high conversion of cumyl alcohol at low cost.

That is, the present invention relates to a process for producing α-methyl styrene, which comprises dehydrating cumyl alcohol in the presence of an activated alumina, wherein a concentration of an organic acid contained in a raw material containing cumyl alcohol is 10 to 1,000 ppm by weight.

### MODE FOR CARRYING OUT THE INVENTION

The dehydration is usually carried out by contacting cumyl alcohol with a catalyst. The reaction can be carried out in a gas phase or a liquid phase using a solvent. The reaction is preferably carried out in a liquid phase from the viewpoint of productivity and energy saving. The solvent should be substantially inert to reactants and products. The solvent may be a substance present in a cumyl alcohol solution to be used. For example, when cumyl alcohol is a mixture with cumene as a product, it is possible to use cumene as a substitute without adding a solvent in particular. As other useful solvents, there can be listed alkanes (e.g. octane, decane, dodecane), mono-cyclic aromatic compounds (e.g. benzene, ethylbenzene, toluene) and the like. The dehydration temperature is usually 50 to 450°C, and preferably 150 to 300°C. In usual, the pressure is advantageously 10 to 10,000 kPa. When the equilibrium of the reaction is considered, it is advantageous that the pressure is set up as low as possible. The dehydration can be advantageously carried out by using a catalyst in the form of a slurry or fixed-bed.

The feature of the present invention is to carry out the reaction in the presence of an activated alumina with allowing to exist the organic acid of 10 to 1000 ppm by weight in the raw material containing cumyl alcohol. The concentration of the organic acids in the raw material is preferably 50 to 1000 ppm by weight. In addition, herein, when a medium such as a solvent(liquid phase reaction) or a diluting gas(gas phase reaction) is contained in the raw material, the concentration means the organic acid concentration in the raw material including the medium. The activity of the activated alumina is improved by allowing to exist the organic acids within the above range, and high conversion of cumyl alcohol can be attained. The organic acid is preferably an organic carboxylic acid, and more preferably at least one selected from the group consisting of formic acid, acetic acid and propionic acid. The existence of the organic acid in a large amount exceeding 1000 ppm by weight is not preferable for problems of corrosion of the apparatus, and the like.

The process of the present invention can be preferably applied to, for example, a dehydration step in production of propylene oxide containing the following steps, but is not limited thereto:
oxidation step; a step of obtaining cumene hydroperoxide by oxidizing cumene;
epoxidation step: a step of obtaining propylene oxide and cumyl alcohol by reacting cumene hydroperoxide contained in a cumene solution with propylene in an excess amount in the presence of an epoxidation catalyst in a liquid phase:
dehydration step: a step of obtaining a -methyl styrene by dehydrating cumyl alcohol obtained in the epoxidation step in the presence of a dehydration catalyst; and
hydrogenation step: a step of converting α -methyl styrene into cumene in the presence of a hydrogenation catalyst and recycling the cumene to the oxidation step as a raw material in the oxidation step.

The oxidation of cumene in the oxidation step is usually conducted by auto-oxidation using an oxygen-containing gas such as air or oxygen-concentrated air. This oxidation may be conducted without use of an additive, or an additive such as an alkali may be used. The reaction temperature is usually from 50 to 200°C , and the reaction pressure is usually between atmospheric pressure and 5 MPa. In the oxidation method in which the additive is used, an alkali metal compound such as NaOH or KOH, an alkaline earth metal compound, an alkali metal carbonate such as Na₂CO₃ or NaHCO₃, ammonia, (NH₄)₂CO₃, an alkali metal ammonium carbonate or the like, is used as the alkali reagent.

As the catalyst used in the epoxidation step, a solid catalyst containing a titanium-containing silicon oxide is preferable from the viewpoint of obtaining the objective product under high yield and high selectivity. As the catalyst, a so-called Ti-silica catalyst containing Ti chemically bonded to silicon oxide, is preferable. For example, a catalyst prepared by supporting a Ti compound on a silica carrier, a catalyst prepared by combining a Ti compound with silicon oxide by a co-precipitation method or sol gel method, zeolite compounds containing Ti, and the like, can be listed.

Cumene hydroperoxide used as the raw material in the epoxidation step, may be a dilute or dense purified material or non-purified material.

The epoxidation is carried out by contacting propylene and cumene hydroperoxide with the catalyst. The reaction is carried out in a liquid phase using a solvent. The solvent should be liquid under a temperature and pressure in the reaction, and substantially inert to the reactants and products. The solvent may be a substance present in a hydroperoxide solution to be used. For example, when cumene hydroperoxide is a mixture with cumene which is a raw material thereof, the cumene can be used as a substitute of a solvent without particularly adding a solvent. Additionally, mono-cyclic aromatic compounds (e.g. benzene, toluene, chlorobenzene, orthodichlorobenzene), alkanes (e.g. octane, decane, dodecane) and the like, can be listed as useful solvents.

The epoxidation temperature is usually from 0 to 200°C, and preferably from 25 to 200°C. The pressure may be a pressure enough to keep the reaction mixture in a liquid condition. In general, the pressure is advantageously from 100 to 10,000 kPa.

The catalyst can be advantageously used in the form of a slurry or fixed bed. In the case of a large-scale industrial operation, a fixed bed is preferably used. In addition, the epoxidation can be conducted by a batch-wise method, semi-continuous method or continuous method.

The molar ratio of propylene to cumene hydroperoxide supplied to the epoxidation step, is preferably 2/1 to 50/1. When the ratio is smaller than 2/1, the efficiency may be deteriorated by lowering of the reaction rate, on the other hand, when the ratio is larger than 50/1, large energy in the recycling is required because the amount of propylene to be recycled becomes bigger.

The dehydration is as described above. It is possible to use hydrogen, as an inert gas, to be used in the next hydrogenation step.

As the hydrogenation catalyst used in the hydrogenation step, a solid catalyst containing a metal of Group 10 or 11 of the Periodic Table, can be listed. Specifically, nickel, palladium, platinum and copper are listed, and among these, palladium and copper are preferable from the viewpoint of suppression of hydrogenation of the aromatic ring and high yield. A copper-based catalyst includes copper, Raney copper, copper-chromium, copper-zinc, copper-chromium-zinc, copper-silica, copper-alumina and the like. A palladium-based catalyst includes palladium-alumina, palladium- silica, palladium- carbon and the like.

The hydrogenation is carried out by contacting α -methyl styrene and hydrogen with the hydrogenation catalyst. The reaction can be carried out in a liquid phase using a solvent or a gas phase. The solvent should be substantially inert to the reactants and products. The solvent may be a substance existing in an α-methyl styrene solution to be used. For example, when α-methyl styrene is a mixture with cumene as a product, it is possible to use cumene as a substitute of the solvent without adding a solvent in particular. As other useful solvents, there can be listed alkanes (e.g. octane, decane, dodecane), mono-cyclic aromatic compounds (e.g. benzene, ethylbenzene, toluene) and the like. The hydrogenation temperature is usually 0 to 500°C, and preferably 30 to 400°C. In usual, the pressure is advantageously 100 to 10,000 kPa. Cumene obtained by hydrogenation of α-methyl styrene, is recycled to the above-described oxidation step as a raw material.

### EXAMPLE

Next, the present invention is explained by Examples.

### Example 1

A cumene solution (containing 200 ppm of formic acid) containing 25% by weight of cumyl alcohol and hydrogen were passed through a fixed bed flow reactor in which an activated alumina was packed, at a rate of 1.6g/minute and 105 Ncc/minute, respectively. At this time, LHSV(Liquid Hourly Space Velocity) was 9/hour, the pressure was 1.0 MPaG, and the temperature was 200°C. The dehydration conversion of cumyl alcohol in the obtained reaction mixture was 97%.

### Example 2

It was carried out in the same manner as in Example 1 except that a cumene solution (containing 130 ppm of formic acid) containing 25% by weight of cumyl alcohol was used. The dehydration conversion of cumyl alcohol in the obtained reaction mixture was 93%.

### Example 3

It was carried out in the same manner as in Example 1 except that a cumene solution (containing 60 ppm of formic acid) containing 25% by weight of cumyl alcohol was used. The dehydration conversion of cumyl alcohol in the obtained reaction mixture was 85%.

### Example 4

It was carried out in the same manner as in Example 1 except that a cumene solution (containing 30 ppm of formic acid) containing 25% by weight of cumyl alcohol was used. The dehydration conversion of cumyl alcohol in the obtained reaction mixture was 74%.

### Comparative Example 1

It was carried out in the same manner as in Example 1 except that a cumene solution (containing 5 ppm of formic acid) containing 25% by weight of cumyl alcohol was used. The dehydration conversion of cumyl alcohol in the obtained reaction mixture was 46%.

### INDUSTRIAL APPLICABILITY

As described above, according to the present invention, there can be provided a process for producing α-methyl styrene, which can attain efficiently high conversion of cumyl alcohol at low cost.

## Claims

1. A process for producing α-methyl styrene, which comprises dehydrating cumyl alcohol in the presence of an activated alumina, wherein a concentration of an organic acid contained in a raw material containing cumyl alcohol is 10 to 1,000 ppm by weight.

2. The process according to claim 1, wherein the organic acid in the raw material is at least one selected from the group consisting of formic acid, acetic acid and propionic acid.

3. The process according to claim 1, wherein the process is a part of a process for producing propylene oxide comprising the following steps:
oxidation step: a step of obtaining cumene hydroperoxide by oxidizing cumene;
epoxidation step: a step of obtaining propylene oxide and cumyl alcohol by reacting cumene hydroperoxide contained in a cumene solution with propylene in an excess amount in the presence of a epoxidation catalyst in a liquid phase;
dehydration step: a step of obtaining α -methyl styrene by dehydrating cumyl alcohol obtained in the epoxidation step in the presence of a dehydration catalyst; and
hydrogenation step: a step of hydrogenating α-methyl styrene in the presence of a hydrogenation catalyst to convert into cumene and recycling it to the oxidation step as a raw material.
